# EUROPEAN PATENT APPLICATION

(11) **EP 4 653 026 A1**
(43) Date of publication of application: **26.11.2025**
(21) Application number: 24770678.1
(22) Date of filing: 06.03.2024
(51) Int. Cl.: A61M 1/14, A61M 1/16

(54) **BLOOD PURIFICATION DEVICE AND BLOOD PURIFICATION SYSTEM**

(30) Priority: 10.03.2023 JP 2023037402
(71) Applicant: Nikkiso Co., Ltd., Tokyo 150-6022 (JP)
(72) Inventor: MENJOH, Yuya, Higashimurayama-shi Tokyo 189-8520 (JP); MOCHIZUKI, Hiroaki, Higashimurayama-shi Tokyo 189-8520 (JP)
(74) Representative: von Hirschhausen, Helge
(86) International application number: PCT/JP2024/008619
(87) International publication number: WO 2024/190572

(57) **Abstract**

This blood purification device 1 is configured by being divided into a plurality of units 3 and 4. At least one unit 3 of the plurality of units 3 and 4 is configured so as to have a storage part 7 for storing calibration data 91 of the unit 3, and when the unit 3 is connected to the other unit 4, the calibration data 91 can be shared with the other unit 4.

## Description

### TECHNICAL FIELD

The present invention relates to a blood purification device and a blood purification system.

### BACKGROUND OF THE INVENTION

An integrated-type blood purification device, which integrally includes a dialysate supply-and-discharge portion to supply and discharge dialysate to and from a blood purifier and an extracorporeal circulation portion to extracorporeally circulate blood of a patient through the blood purifier, is known as a conventional blood purification device to perform blood purification treatment.

Prior art document information related to the invention of the present application includes Patent Literature 1.

### CITATION LIST

### PATENT LITERATURES

Patent Literature 1: Japanese Unexamined Patent Application Publication (Translation of PCT Application) No. 2016-502911

### SUMMARY OF THE INVENTION

The present inventors have been studying a separable-type blood purification device composed of plural separate units to improve ease of maintenance. The blood purification device composed of plural separate units allows for swap-out maintenance where a unit requiring maintenance is replaced with a unit that has already been maintained, and it is thereby possible to significantly improve ease of maintenance. However, the problem is that when the unit is replaced, the sensors or actuators need to be calibrated again and it thus takes time to set up.

Therefore, it is an object of the invention to provide a separable-type blood purification device that can shorten the setup time.

A blood purification device in an embodiment of the invention comprises:
a plurality of separate units,
wherein among the plurality of units, at least one unit comprises a storage unit to store calibration data of the unit, and when the unit is connected to another unit, the calibration data can be shared with the other unit.

A blood purification system in an embodiment of the invention comprises:
a blood purification device comprising a plurality of separate units; and
a network device that is configured to be able to communicate with at least one unit among the plurality of units and comprises a storage unit to store calibration data of a predetermined unit thereamong,
wherein the at least one unit of the blood purification device comprises a control unit that, when the unit is connected to another unit, acquires the calibration data of the connected other unit through the network device.

### Advantageous Effects of the Invention

According to the invention, it is possible to provide a blood purification device and a blood purification system that can shorten the setup time.

### BRIEF DESCRIPTION OF DRAWINGS

Fig. 1A is a diagram illustrating the appearance of a blood purification device in an embodiment of the present invention and is a perspective view when an extracorporeal circulation unit is attached to a dialysate supply-and-discharge unit.
Fig. 1B is a diagram showing the appearance of the blood purification device in the embodiment of the invention and is a perspective view when the extracorporeal circulation unit is detached from the dialysate supply-and-discharge unit.
Fig. 2 is a schematic configuration diagram illustrating the blood purification device in the embodiment of the invention.
Fig.3 is an explanatory diagram illustrating details of a control unit and a storage unit in each unit.
Fig. 4 is a flowchart showing a control flow when setting up the blood purification device.
FIG. 5 is a flowchart showing a control flow of calibration processing.
Fig. 6 is a schematic configuration diagram illustrating a blood purification system in an embodiment of the invention.

### DETAILED DESCRIPTION OF THE INVENTION

### Embodiment

An embodiment of the invention will be described below in conjunction with the appended drawings.

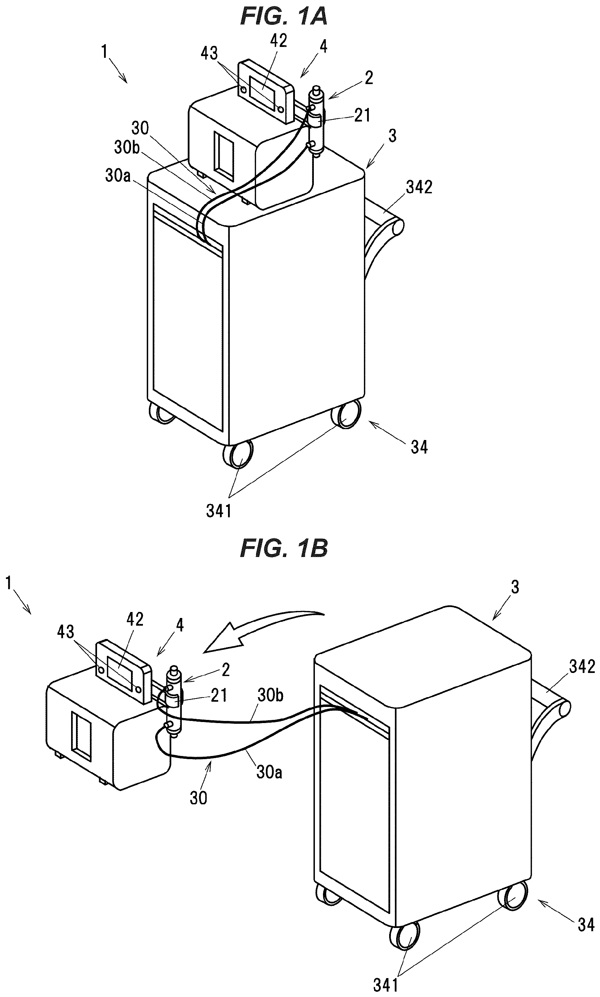

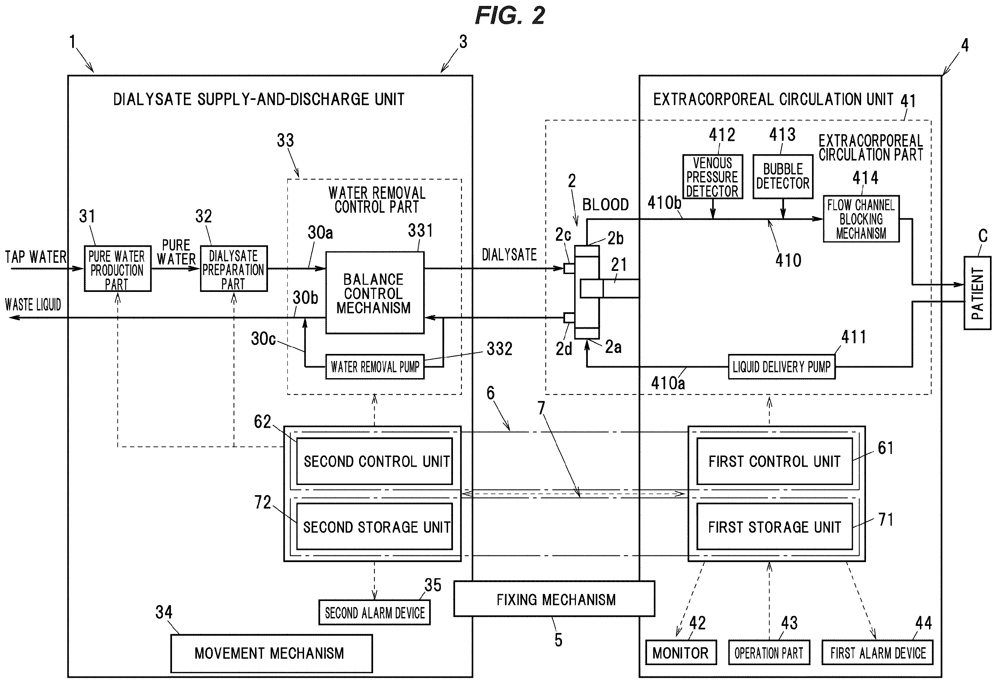

Fig. 1A is a diagram illustrating the appearance of a blood purification device in the present embodiment and is a perspective view when an extracorporeal circulation unit is attached to a dialysate supply-and-discharge unit. Fig. 1B is a perspective view when the extracorporeal circulation unit is detached from the dialysate supply-and-discharge unit. Fig. 2 is a schematic configuration diagram illustrating the blood purification device in the present embodiment.

As shown in Figs. 1A, 1B and 2, a blood purification device 1 is a device to perform blood purification treatment through a blood purifier 2 and includes a dialysate supply-and-discharge unit 3 that supplies a dialysate to the blood purifier 2 and discharges a waste liquid from the blood purifier 2, and an extracorporeal circulation unit 4 including an extracorporeal circulation part 41 to extracorporeally circulate blood of a patient through the blood purifier 2. That is, the blood purification device 1 in the present embodiment is composed of plural separate units. The case where the device is separated into two units, the dialysate supply-and-discharge unit 3 and the extracorporeal circulation unit 4, will be described here. However, the device may have, e.g., a three-unit configuration with a pure water production part 31 (described later) as a separate unit, and the number of separate units is not limited.

### Blood purifier 2

The blood purifier 2 is also called a dialyzer and includes a blood purification membrane (a hollow-fiber hemodialysis membrane or hemodialysis filtration membrane, or a flat hemodialysis membrane or hemofiltration membrane) thereinside. The blood purifier 2 has a blood inlet 2a to introduce blood and a blood outlet 2b to discharge the introduced blood, as well as a dialysate inlet 2c to introduce dialysate and a dialysate outlet 2d to discharge the introduced dialysate. In the dialysis device 2, blood is purified by bringing the blood into contact with the dialysate through the blood purification membrane. In the present embodiment, the blood purifier 2 is detachably attached to the extracorporeal circulation unit 4 through a blood purifier fixing jig 21. In this regard, however, the position to attach the blood purifier 2 is not limited thereto, and the blood purifier 2 may be attached to the dialysate supply-and-discharge unit 3 or a dedicated stand, etc.

### Dialysate supply-and-discharge unit 3

The dialysate supply-and-discharge unit 3 has the pure water production part 31, a dialysate preparation part 32, and a water removal control part 33. The dialysate supply-and-discharge unit 3 also has a dialysate supply-and-discharge unit-side tube 30 to convey the dialysate and the waste liquid. The dialysate supply-and-discharge unit-side tube 30 has a supply-side tube 30a to supply the dialysate to the blood purifier 2 and a discharge-side tube 30b to discharge the waste liquid from the blood purifier 2.

The pure water production part 31 is provided on the supply-side tube 30a and is configured, e.g., to produce pure water (RO water) by using a reverse osmosis membrane (RO membrane) and removing impurities from tap water supplied externally through the supply-side tube 30a. In this regard, the pure water production part 31 can be omitted, and the configuration may be such that pure water is externally supplied to the dialysate supply-and-discharge unit 3.

The dialysate preparation part 32 is provided on the supply-side tube 30a and is configured to prepare dialysate from pure water supplied from the pure water production part 31 through the supply-side tube 30a and a dialysate concentrate made of a concentrated solution or powder. In this regard, the dialysate preparation part 32 can also be omitted, and the configuration may be such that, e.g., dialysate is supplied to the dialysate supply-and-discharge unit 3 from an external dialysate supply device or bag, etc.

The water removal control part 33 controls the amount of water removed from blood, and has a balance control mechanism 331 that delivers liquids so that the amount of the dialysate supplied to the blood purifier 2 is equal to the amount of waste liquid, and a water removal pump 332. The balance control mechanism 331 is placed over the supply-side tube 30a from the dialysate preparation part 32 to the blood purifier 2 and the discharge-side tube 30b extending from the blood purifier 2 and drawn into the dialysate supply-and-discharge unit 3. The balance control mechanism 331 is constructed from, e.g., a dual pump which maintains equality of the supplied liquid and the waste liquid by reciprocating movement of a plunger between two pump chambers with the same volume, etc.

A bypass tube 30c which bypasses the balance control mechanism 331 is provided on the discharge-side tube 30b, and the water removal pump 332 is arranged on the bypass tube 30c. By driving the water removal pump 332, the amount of the waste liquid is increased to more than the amount of the dialysate supplied to the blood purifier 2 and water is removed from the blood. The amount of water removed from the blood can be controlled by controlling drive of the water removal pump 332. In this regard, the specific structure of the water removal control part 33 is not limited to that shown in the drawing, and the configuration may be such that, e.g., the bypass tube 30c and the water removal pump 332 are omitted, the flow rates of the dialysate and the waste liquid are controlled separately by the balance control mechanism 331, and the removed water amount is controlled based on a difference in the flow rate between the dialysate and the waste liquid.

The supply-side tube 30a and the discharge-side tube 30b located on the blood purifier 2 side relative to the water removal control part 33 are drawn to the outside of the dialysate supply-and-discharge unit 3, and ends of the drawn-out supply-side tube 30a and discharge-side tube 30b are connected to the blood purifier 2. In more particular, an end (downstream end) of the supply-side tube 30a is connected to the dialysate inlet 2c of the blood purifier 2, and an end (upstream end) of the discharge-side tube 30b is connected to the dialysate outlet 2d of the blood purifier 2. The discharge-side tube 30b extending from the water removal control part 33 toward the side opposite to the blood purifier 2 (toward the downstream side in the waste liquid flow) is drawn out to the outside of the dialysate supply-and-discharge unit 3, and the waste liquid is discharged to the outside of the dialysate supply-and-discharge unit 3. However, the configuration is not limited thereto, and a waste liquid tank to store the waste liquid may be provided in the dialysate supply-and-discharge unit 3.

As described above, in the present embodiment, tubes to convey the dialysate and the waste liquid (in this example, the supply-side tube 30a and the discharge-side tube 30b) are configured to directly connect the dialysate supply-and-discharge unit 3 to the blood purifier 2 without interposition of the extracorporeal circulation unit 4. In other words, tubes to convey the dialysate and the waste liquid are not provided in the extracorporeal circulation unit 4. This eliminates the need for a liquid tube connecting the dialysate supply-and-discharge unit 3 to the extracorporeal circulation unit 4, ease of handling is improved and the cost is reduced by simplifying the structure. The tube to convey the dialysate or the waste liquid (the dialysate supply-and-discharge unit-side tube 30) is desirably a fixed tube and is cleaned, sanitized and used repeatedly since the running cost is very high if disposable.

Although it is not shown in the drawing, the dialysate supply-and-discharge unit 3 may further include, e.g., a mechanism to adjust temperature of the dialysate, a deaeration mechanism to remove dissolved oxygen in the dialysate, a filter to remove fine particulates (endotoxin, etc.) in the dialysate, a blood leak detector to detect blood leakage, or a mechanism to measure solute concentrations in the dialysate after passing through the blood purifier 2 by irradiation with ultraviolet rays and determine dialysis efficiency. In addition, the dialysate supply-and-discharge unit 3 may further include a dialysate regeneration part to regenerate used dialysate. The dialysate regeneration part is configured to include, e.g., an adsorber that adsorbs and removes ammonia, and an injection part to inject an infusion solution to adjust the components of the dialysate.

The dialysate supply-and-discharge unit 3 is heavier than the extracorporeal circulation unit 4 (described later). Therefore, to easily move the dialysate supply-and-discharge unit 3 and increase convenience and ease of handling, the dialysate supply-and-discharge unit 3 desirably includes a movement mechanism 34 to move the dialysate supply-and-discharge unit 3 on a floor. Providing the movement mechanism 34 also makes it easier to perform swap-out maintenance (described later), which further improves ease of maintenance. In the present embodiment, casters 341 are provided on the bottom of the dialysate supply-and-discharge unit 3, and a handle 342 to hold when moving the dialysate supply-and-discharge unit 3 is provided on a side portion of the dialysate supply-and-discharge unit 3 (see Figs. 1A and 1B). However, the specific structure of the movement mechanism 34 is not limited thereto.

Although it is not shown in the drawing, power is externally supplied to the dialysate supply-and-discharge unit 3. In this regard, the dialysate supply-and-discharge unit 3 may be configured to include a backup power supply such as battery so that power is supplied from the backup power supply when the external power supply is interrupted during blood purification treatment. Furthermore, it may be configured to supply power from the dialysate supply-and-discharge unit 3 to the extracorporeal circulation unit 4.

The dialysate supply-and-discharge unit 3 also includes a second control unit 62 for controlling the dialysate supply-and-discharge unit 3, and a second storage unit 72. The details of the second control unit 62 and the second storage unit 72 will be described later. The dialysate supply-and-discharge unit 3 also has a second alarm device 35 to issue a notification of detection of an abnormality, etc. The second alarm device 35 has, e.g., a light-emitting device or a buzzer, etc., and issues an alarm by light or sound.

### Extracorporeal circulation unit 4

The extracorporeal circulation unit 4 has the extracorporeal circulation part 41 having a blood tube (blood circuit) 410 capable of extracorporeally circulating blood of a patient C.

The blood tube 410 is formed of, e.g., a flexible tube, etc. The blood tube 410 has an artery-side blood tube 410a through which the blood taken from a blood vessel of the patient C is guided to the blood inlet 2a of the blood purifier 2, and a vein-side blood tube 410b through which the blood discharged from the blood outlet 2b of the blood purifier 2 returns to the patient C. In the present embodiment, the blood tube 410 is detachably provided in the extracorporeal circulation unit 4. This allows the blood tube 410 to be disposable, which enhances ease of maintenance. The blood tube 410 is not shown in Figs. 1A and 1B.

The extracorporeal circulation part 41 has a liquid delivery pump 411 that is placed on the artery-side blood tube 410a and circulates the blood. The liquid delivery pump 411 is constructed from, e.g., a peristaltic pump which forces the blood to flow toward the blood purifier 2 by squeezing a tube. The extracorporeal circulation part 41 also has a venous pressure detector 412 which is placed on the vein-side blood tube 410b to measure pressure of the blood flowing through the blood tube 410, and a bubble detector 413 which detects air bubbles in the blood. The bubble detector 413 is configured to have, e.g., a pair of ultrasonic vibration elements (a means for generating oscillation and a means for receiving oscillation) formed of piezoelectric elements. A flow channel blocking mechanism 414 is also placed on the vein-side blood tube 410b and closes the vein-side blood tube 410b to stop the extracorporeal circulation of the blood when an abnormality occurs, such as when air bubbles are detected by the bubble detector 413.

In the blood purification device 1 of the present embodiment, the extracorporeal circulation 4 is configured as a separate unit from the dialysate supply-and-discharge unit 3. The extracorporeal circulation unit 4 includes a first control unit 61 capable of controlling the dialysate supply-and-discharge unit 3 and the extracorporeal circulation unit 4, and a first storage unit 71. The details of the first control unit 61 and the first storage unit 71 will be described later.

The extracorporeal circulation unit 4 is configured to be detachable from the dialysate supply-and-discharge unit 3 (see Figs. 1A and 1B). This allows use in various layouts according to the situation, e.g., a layout in which only the extracorporeal circulation unit 4 is placed close to the patient C and the dialysate supply-and-discharge unit 3 is placed at a distant position. Although the example in which the extracorporeal circulation unit 4 is attached on the top of the dialysate supply-and-discharge unit 3 is shown in Figs. 1A and 1B, it is not limited thereto. For example, the two units 3 and 4 may be arranged side by side on the left and right or arranged front and back.

The blood purification device 1 also includes a fixing mechanism 5 that fixes the extracorporeal circulation unit 4 and the dialysate supply-and-discharge unit 3 to each other when the extracorporeal circulation unit 4 is attached to the dialysate supply-and-discharge unit 3. This suppresses falling of the extracorporeal circulation unit 4 from the dialysate supply-and-discharge unit 3 when moving the blood purification device 1 or even when a person, etc. unintentionally bumps into the blood purification device 1. The specific structure of the fixing mechanism 5 is not limited, and it is possible to use, e.g., a one-touch fixing mechanism 5 that uses a latch mechanism, etc. The fixing mechanism 5 is not shown in Figs. 1A and 1B.

The extracorporeal circulation unit 4 also has a monitor 42 to display progress of dialysis and operation parts 43 to perform an operation related to blood purification treatment. The monitor 42 is provided so as to protrude from the top of the extracorporeal circulation unit 4 in the present embodiment as shown in Figs. 1A and 1B, but the position or installation layout of the monitor 42 is not limited thereto. For example, the monitor 42 which is large in size may be provided so as to cover the entire surface of the extracorporeal circulation unit 4. In addition, although the operation parts 43 are provided on both sides of the monitor 42 in the present embodiment, the positions or layout of the operation parts 43 are not limited thereto. In addition, the monitor 42 may be configured as a touch panel so that the monitor 42 also serves as the operation part 43. By providing the monitor 42 and the operation parts 43 on the extracorporeal circulation unit 4 which is relatively lightweight and placed close to the patient C, the patient C can easily check the progress of treatment or can easily operate the unit and convenience can be improved.

The monitor 42 and the operation part 43 may be configured as separate components from the extracorporeal circulation unit 4 and can be configured to communicate with the first control unit 61 of the extracorporeal circulation unit 4 for display and operation. In this case, the monitor 42 and the operation part 43 can be composed of, e.g., a dedicated operation terminal, or a smartphone or tablet, etc. Furthermore, the monitor 42 and the operating part 43 may be mounted on the dialysate supply-and-discharge unit 3.

The extracorporeal circulation unit 4 also has a first alarm device 44 to issue a notification of detection of an abnormality, etc. The first alarm device 44 has, e.g., a light-emitting device or a buzzer, etc., and issues an alarm by light or sound.

### Control unit 6 and Storage unit 7 of each unit 3, 4

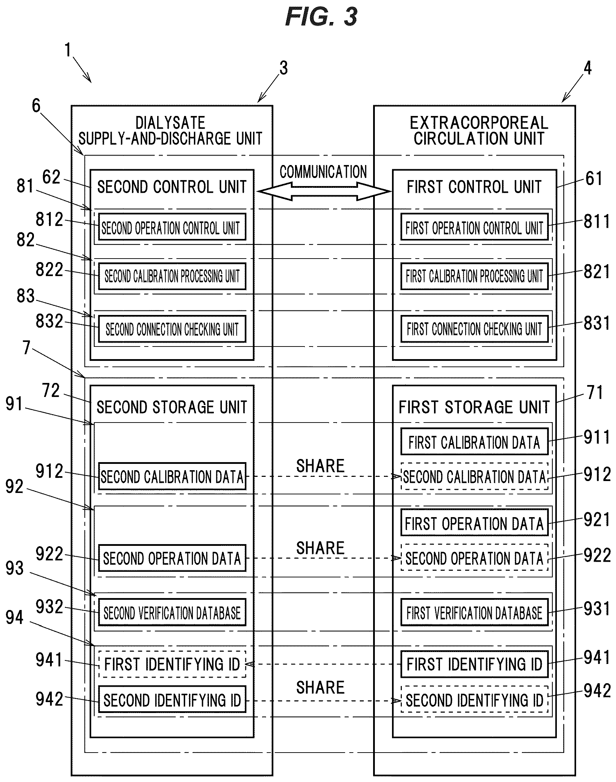

As shown in Fig. 3 , the blood purification device 1 of the present embodiment is configured such that among the plural units 3, 4 constituting the blood purification device 1, at least one unit (the dialysate supply-and-discharge unit 3 in this example) has a storage unit 7 to store calibration data 91 and operation data 92 of the unit 3, and when the unit 3 is connected to another unit (the extracorporeal circulation unit 4 in this example), the calibration data 91 and the operation data 92 can be shared with the other unit 4. In the present embodiment, each of the two units 3 and 4 includes a control unit 6 and the storage unit 7. Hereinafter, the control unit 6 and the storage unit 7 mounted on the extracorporeal circulation unit 4 will be referred to as the first control unit 61 and the first storage unit 71, and the control unit 6 and storage unit 7 mounted on the dialysate supply-and-discharge unit 3 will be referred to as the second control unit 62 and the second memory unit 72. Each of the control units 61 and 62 is realized by appropriately combining an arithmetic element such as CPU, a memory, software, interface and a communication unit, etc. Each of the storage units 71 and 72 is realized by using a predetermined storage region of a memory or a storage device. In the present embodiment, the storage units 71 and 72 are formed using non-volatile memories.

Each of the control units 61 and 62 of the two units 3 and 4 has an operation control unit 81, a calibration processing unit 82, and a connection checking unit 83. Hereinafter, the units mounted on the extracorporeal circulation unit 4 will be respectively referred to as a first operation control unit 811, a first calibration processing unit 821, and a first connection checking unit 831, and the units mounted on the dialysate supply-and-discharge unit 3 will be respectively referred to as a second operation control unit 812, a second calibration processing unit 822, and a second connection checking unit 832.

Each of the storage units 71 and 72 of the two units 3 and 4 stores the calibration data 91, the operation data 92, a verification database 93, and an identifying ID 94 of the own unit. The calibration data 91 is data that includes information related to calibration such as calibration data for various sensors, actuators, pumps, etc. included in the own unit. The operation data 92 is data that includes information of the operation of the own unit, and more specifically, includes various information related to the operation, e.g., information indicating that the sanitization process has been completed, or information indicating that an operation of filling a tube with dialysate has been performed. The verification database 93 is a database to determine whether a connected unit is configured to be connectable to the own unit, and is a database in which the models, etc. of units connectable to the own unit are stored. The identifying ID 94 includes information of the own unit, such as the individual identification number, model, serial number, software version, types of performable treatment, and whether or not having any attachable optional devices. Hereafter, the data stored in the first storage unit 71 of the extracorporeal circulation unit 4 will be respectively referred to as first calibration data 911, first operation data 921, a first verification database 931, and a first identifying ID 941, and the data stored in the second storage unit 72 of the dialysate supply-and-discharge unit 3 will be respectively referred to as second calibration data 912, second operation data 922, a second verification database 932, and a second identifying ID 942.

The first control unit 61 of the extracorporeal circulation unit 4 serves as the main control unit of the blood purification device 1. The first operation control unit 811 controls the extracorporeal circulation unit 4, and also communicates with the second control unit 62 of the dialysate supply-and-discharge unit 3 and controls the dialysate supply-and-discharge unit 3 through the second control unit 62. The first operation control unit 811 stores its control contents (information related to the operation) as the first operation data 921 in the first storage unit 71. The first operation data 921 is data that includes the control contents (information related to the operation) for the extracorporeal circulation unit 4. The first calibration data 911 is data that includes information related to calibration of the extracorporeal circulation unit 4.

The first operation control unit 811 acquires the second calibration data 912 and the second operation data 922 of the dialysate supply-and-discharge unit 3, and controls the dialysate supply-and-discharge unit 3 based on the acquired (i.e., shared) second calibration data 912 and second operation data 922. The second operation data 922 is data that includes the contents of control by the second control unit 62, i.e., the control contents (information related to the operation) for the dialysate supply-and-discharge unit 3. The second calibration data 912 is data that includes information related to calibration of the dialysate supply-and-discharge unit 3. Sharing the second calibration data 912 eliminates the need to perform calibration each time a new dialysate supply-and-discharge unit 3 is connected, hence, the setup time can be shortened. Meanwhile, sharing the second operation data 922 allows the device to be used in such a manner that, e.g., the dialysate supply-and-discharge unit 3 after performing the sanitization process and preparation for blood purification treatment is connected to the extracorporeal circulation unit 4 to allow prompt start of the treatment, hence, convenience is improved. The timing of sharing the second calibration data 912 and the second operation data 922 is not particularly limited. For example, the data may be shared automatically when the extracorporeal circulation unit 4 is activated, or the data may be shared when a data sharing operation is performed by a user.

The first calibration processing unit 821 performs calibration processing, such as calibration of various sensors, actuators, and pumps of the extracorporeal circulation unit 4, and stores the calibration results as the first calibration data 911 in the first storage unit 71.

The first connection checking unit 831 identifies the connected dialysate supply-and-discharge unit 3 and determines whether or not it is configured to be connectable to the own unit. The first connection checking unit 831 communicates with the second control unit 62, acquires the second identifying ID 942 of the dialysate supply-and-discharge unit 3, and refers to the first verification database 931 to determine whether the connected dialysate supply-and-discharge unit 3 is configured to be connectable to the own unit. When, as a result of the determination, the dialysate supply-and-discharge unit 3 is not a connectable unit, the first connection checking unit 831, e.g., issues an alarm using the first alarm device 44 or displays a warning message on the monitor 42. Although whether or not the unit is connectable to the own unit is determined by referring to the first verification database 931 in the present embodiment, it is not limited thereto. The first connection checking unit 831 may be configured such that, e.g., information allowing to identify the type of unit or whether or not having any attachable optional devices, etc. (information allowing to determine whether connectable to the own unit) is included in the second identifying ID 942 and whether or not connectable to the own unit is determined based on the information included in the second identifying ID 942. In this case, the first verification database 931 can be omitted.

The second control unit 62 of the dialysate supply-and-discharge unit 3 is a sub-control unit. The second operation control unit 812 controls the dialysate supply-and-discharge unit 3 in accordance with instructions from the first operation control unit 811. In this regard, it is possible to control the dialysate supply-and-discharge unit 3 directly by the first control unit of the extracorporeal circulation unit 4, but in this case, the number of control lines connecting the two units 3, 4 becomes huge and the connection structure of the two units 3, 4 becomes very complicated. By providing the control unit 6 (the second control unit 62) also on the dialysate supply-and-discharge unit 3, the number of control lines connecting the two units 3 and 4 can be reduced and the connection structure of the two units 3 and 4 can be simplified.

The second operation control unit 812 is desirably configured to allow the dialysate supply-and-discharge unit 3 to operate even individually (operate also in a state of being detached from the extracorporeal circulation unit 4). This makes it possible to clean and sanitize the dialysate supply-and-discharge unit 3 alone. The second operation control unit 812 stores its control contents (information related to the operation) as the second operation data 922 in the second storage unit 72.

The second calibration processing unit 822 performs calibration processing, such as calibration of various sensors, actuators, and pumps of the dialysate supply-and-discharge unit 3, and stores the calibration results as the second calibration data 912 in the second storage unit 72.

The second connection checking unit 832 identifies the connected extracorporeal circulation unit 4 and determines whether or not it is configured to be connectable to the own unit. The second connection checking unit 832 communicates with the first control unit 61, acquires the first identifying ID 941 of the extracorporeal circulation unit 4, and refers to the second verification database 932 to determine whether the connected extracorporeal circulation unit 4 is configured to be connectable to the own unit. When, as a result of the determination, the extracorporeal circulation unit 4 is not a connectable unit, the second connection checking unit 832, e.g., issues an alarm using the second alarm device 35. In this regard, the second connection checking unit 832 may be configured such that, e.g., information allowing to identify the type of unit or whether or not having any attachable optional devices, etc. (information allowing to determine whether connectable to the own unit) is included in the first identifying ID 941 and whether or not connectable to the own unit is determined based on the information included in the first identifying ID 941. In this case, the second verification database 932 can be omitted.

In this way, in the present embodiment, whether or not the unit is connectable is determined by both the first control unit 61, which is the main control unit, and the second control unit 62, which is the sub-control unit, and this double-check suppresses connection in inappropriate combinations. However, the configuration is not limited thereto and may such that whether or not the unit is connectable is determined by, e.g., only the first control unit 61 which is the main control unit.

### Control flow

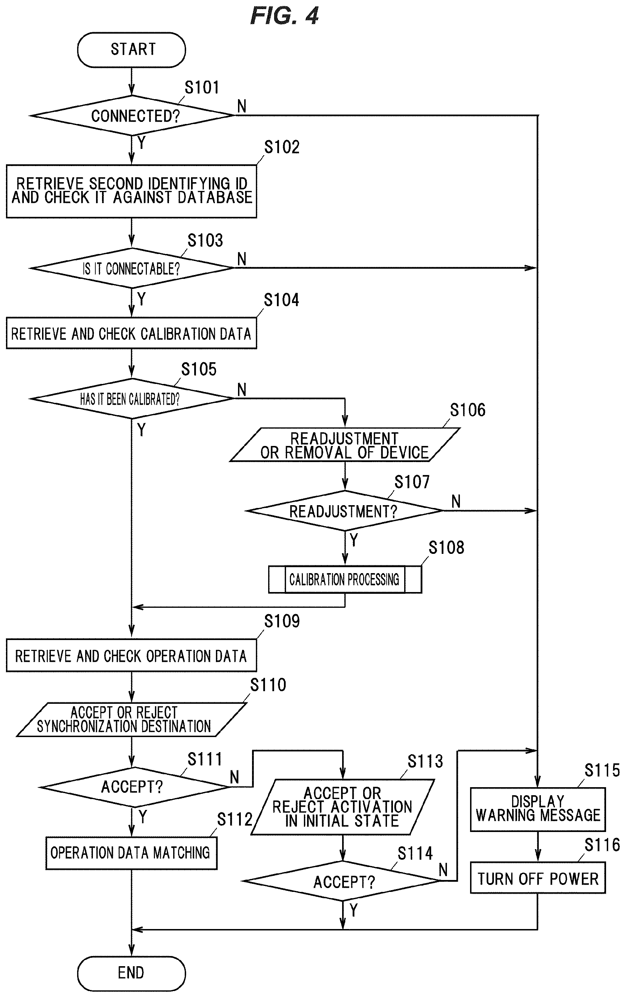

Fig. 4 shows a control flow when setting up the blood purification device 1. The first control unit 61 performs the control flow shown in Fig. 4 when, e.g., the blood purification device 1 is turned on. As shown in Fig. 4, first, in Step S101, the first connection checking unit 831 determines whether or not the dialysate supply-and-discharge unit 3 is connected. In Step S101, whether or not the dialysate supply-and-discharge unit 3 is connected can be determined based on, e.g., whether or not successfully communicating with the second control unit 62. When the determination made in Step S101 is NO, a warning message is displayed on the monitor 42 in Step S115, and the blood purification device 1 is then turned off in Step S116 to end the process.

When the determination made in Step S101 is YES, then in Step S102, the first connection checking unit 831 communicates with the second control unit 62 of the dialysate supply-and-discharge unit 3, acquires the second identifying ID 942 and refers to the first verification database 931 to determine whether the connected dialysate supply-and-discharge unit 3 is configured to be connectable to the own unit. Then, in Step S103, it is determined whether the result of the verification is that the dialysate supply-and-discharge unit 3 is configured to be connectable to the own unit. When the determination made in Step S103 is NO, a warning message is displayed on the monitor 42 in Step S115, and the blood purification device 1 is then turned off in Step S116 to end the process. In Step S116, instead of automatically turning off the blood purification device 1, e.g., a message prompting to turn off the power may be displayed on the monitor 42 to prompt the user to turn off the power.

When the determination made in Step S103 is YES, then in Step S104, the first operation control unit 811 acquires the second calibration data 912 of the dialysate supply-and-discharge unit 3 and checks the details of the calibration of the dialysate supply-and-discharge unit 3 based on the second calibration data 912. Then, in Step S105, it is determined whether the result of the check is that the calibration has been completed. When the determination made in Step S105 is YES, the process proceeds to Step S109. When the determination made in Step S105 is NO, then in Step S106, an input screen to select Readjustment or Removal of the device is displayed on the monitor 42, and a selection input by the user is received. Then, in Step S107, it is determined whether the input is Readjustment. When the determination made in Step S107 is NO, i.e., when Removal of the device is selected, a warning message is displayed on the monitor 42 in Step S115, and the blood purification device 1 is then turned off in Step S116 to end the process. When the determination made in Step S107 is YES, i.e., when Readjustment is selected, calibration processing is performed in Step S108.

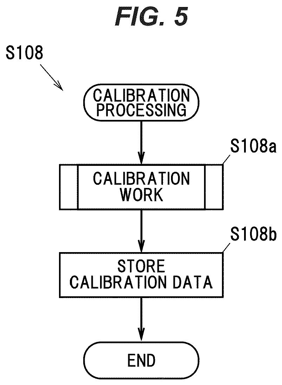

In the calibration processing in Step S108, the second control unit 62 performs the control flow shown in Fig. 5. The second calibration processing unit 822 first performs calibration work such as calibration of various sensors, actuators, pumps, etc., of the dialysate supply-and-discharge unit 3 in accordance with a preset calibration procedure in Step S108a, and stores the results of the calibration work as the second calibration data 912 in the second storage unit 72 in Step S108b. After that, the process returns and proceeds to Step S109 in Fig. 4.

In Step S109, the first operation control unit 811 acquires the second operation data 922 of the dialysate supply-and-discharge unit 3, and checks the details of the operation of the dialysate supply-and-discharge unit 3 based on the second operation data 922. Then, in Step S110, an input screen to select Accept or Reject synchronization destination (whether to synchronize the operation or not) is displayed on the monitor 42, and a selection input by the user is received. Then, in Step S111, it is determined whether the input is Accept. When the determination made in Step S111 is YES, the operation data matching (operation data synchronization) is performed in Step S112, and the setup process then ends. When the determination made in Step S111 is NO, i.e., when Reject is selected, an input screen to select Accept or Reject activation in the initial state is displayed on the monitor 42 in Step S113, and a selection input by the user is received. Then, in Step S114, it is determined whether the input is Accept. When the determination made in Step S114 is YES, the setup process ends without synchronizing the operation. When the determination made in Step S114 is NO, i.e., when Reject is selected, a warning message is displayed on the monitor 42 in Step S115, and the blood purification device 1 is then turned off in Step S116 to end the process.

Although the case where the blood purification device 1 is composed of two units, the extracorporeal circulation unit 4 and the dialysate supply-and-discharge unit 3, has been described here, the number of units is not limited thereto and the device may be composed of not less than three units. In addition, although the case where the extracorporeal circulation unit 4 is the main unit and the dialysate supply-and-discharge unit 3 is the sub-unit has been described here, it is not limited thereto. When, e.g., the dialysate supply-and-discharge unit 3 is the main unit, it is preferable to configure such that the first calibration data 911, which is information related to the calibration of the extracorporeal circulation unit 4, can be shared with the dialysate supply-and-discharge unit 3. In other words, at least information related to calibration of other units should be shared with the main unit.

### Functions and Effects of the embodiment

As described above, the blood purification device 1 in the present embodiment is configured such that among the plural units 3, 4 constituting the blood purification device 1, at least one unit (the dialysate supply-and-discharge unit 3 in this example) has the storage unit 7 to store the calibration data 91 and the operation data 92 of the unit 3, and when the unit 3 is connected to another unit (the extracorporeal circulation unit 4 in this example), the calibration data 91 and the operation data 92 can be shared with the other unit 4.

By storing the calibration data 91 in the storage unit 7 of the own unit and making it possible to share it with the other unit, it is possible to omit the calibration work during the setup even when, e.g., replacing with a new dialysate supply-and-discharge unit 3, thereby shortening the setup time. In addition, sharing the operation data 92 makes it possible, e.g., to prepare for treatment in advance using the dialysate supply-and-discharge unit 3 alone and promptly start the treatment by connecting the dialysate supply-and-discharge unit 3 to the extracorporeal circulation unit 4, hence, convenience is improved.

### Other embodiments

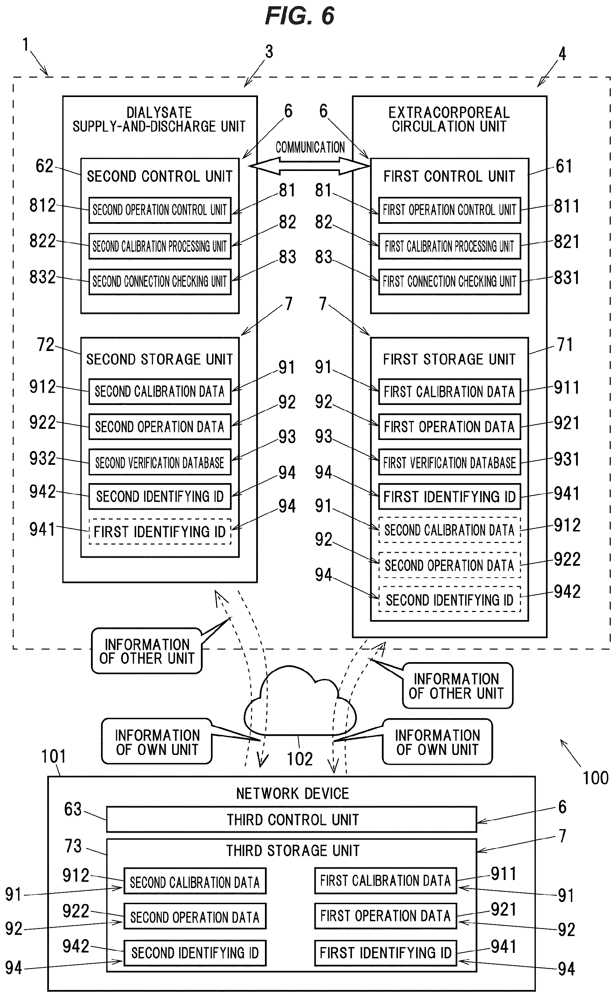

The case where the calibration data 91 and the operation data 92 are stored in the storage unit 7 of the own unit has been described in the above embodiment, but the configuration may be such that the calibration data 91 and the operation data 92 are stored in an external network device 101 such as a server and the units share the calibration data 91 and the operation data 92 through the network device 101 as shown in Fig. 6. Hereinafter, the entire system including the blood purification device 1 and the network device 101 will be referred to as a blood purification system 100.

The network device 101 is configured to be able to intercommunicate with plural units (the extracorporeal circulation unit 4 and the dialysate supply-and-discharge unit 3) constituting the blood purification device 1 through a network 102 such as the Internet. The network device 101 has a third control unit 101a and a third storage unit 101b. A third control unit 63 is realized by appropriately combining an arithmetic element such as CPU, a memory, software, interface and a communication unit, etc. A third storage unit 73 is realized by using a predetermined storage region of a memory or a storage device.

The third control unit 63 communicates with the control units 61 and 62 of the units 3 and 4 of the blood purification device 1 and, e.g., controls the transmission and reception of various data. In the blood purification system 100, the operation control unit 81 and the calibration processing unit 82 of each unit 3, 4 transmit (upload) the operation data 92 and the calibration data 91 of the own unit, together with the identifying ID 93, to the network device 101. The third control unit 63 associates the received operation data 92 and calibration data 91 with the identifying ID 93 and stores in the third storage unit 73.

The operation control unit 81 of each unit 3, 4 acquires the calibration data 91 and the operation data 92 of the other unit connected to the own unit, through the network device 101. This allows the calibration data 91 and the operation data 92 to be shared, hence, it is possible to shorten the setup time and improve convenience in the same manner as the embodiment described above. The connection checking unit 83 of each unit 3, 4 may acquire the identifying ID 93 of the other unit connected to the own unit through the network device 101 or through direct communication between the control units 61 and 62.

Although Fig. 6 shows the case where the second calibration data 91 and the second operation data 92 are also stored in the second storage unit 72 of the dialysate supply-and-discharge unit 3 which is a sub-unit, it is not limited thereto. The configuration may be such that the second calibration data 91 and the second operation data 92 are stored in only the network device 101 without storing the second calibration data 91 and the second operation data 92 in the dialysate supply-and-discharge unit 3. The same applies to the extracorporeal circulation unit 4. In other words, the network device 101 may be treated as an external storage device for each of the units 3 and 4.

In addition, plural blood purification devices 1 may be connected to one network device 101. This makes it possible to manage the calibration data 91, etc., of the plural blood purification devices 1 all at once, which reduces the time and effort required for management.

Furthermore, the connection checking unit 83 may be omitted in each unit 3, 4 of the blood purification device 1, with the network device 101 having a function of checking whether or not the connected units 3, 4 are connectable to each other. In this case, the verification database 93 is installed in the third storage unit 73 of the network device 101, and the third control unit 63 refers to the verification database 93 and, based on the identifying ID 94 of each connected unit 3, 4, determines whether or not connection is possible (whether or not the combination is inappropriate).

In addition, in case that the operation data 92 is not shared, the network device 101 may be configured to be capable of communicating only with the extracorporeal circulation unit 4 which is the main unit. In this case, the extracorporeal circulation unit 4 acquires the identifying ID 93 of the connected dialysate supply-and-discharge unit 3 and transmits the acquired identifying ID 93 to the network device 101. In the third storage unit 73 of the network device 101, the results of calibration performed in advance at the time of shipment from factory, which are associated with the identifying ID 93, are stored as the calibration data 91. The third control unit 63 transmits the calibration data 91 corresponding to the received identifying ID 93, to the extracorporeal circulation unit 4. This allows the calibration data 91 of the dialysate supply-and-discharge unit 3 to be shared. When calibration of the dialysate supply-and-discharge unit 3 is performed again, the calibration data 91 obtained by the calibration may be uploaded to the network device 101 by the extracorporeal circulation unit 4. In this way, the network device 101 only needs to be configured to be able to communicate with at least one of the plural units 3 and 4 constituting the blood purification device 1.

### Modifications

Although not mentioned in the above embodiment, each unit 3, 4 of the blood purification device 1 may be configured to hold connection history data, which is information of the history of the identifying IDs 93 of other units connected to the own unit. This allows an administrator to refer to the connection history of each unit 3, 4 and use it as a reference when, e.g., investigating the cause of a malfunction, etc.

In addition, each unit 3, 4 of the blood purification device 1 may be configured such that the storage unit 7 of the own unit stores unit information data which is information indicating the status of the own unit such as the replacement date and hours of use of consumables included in the own unit, total operating hours of the own unit, and operating hours since the last maintenance. This makes it possible to easily determine whether or not replacement of consumables or maintenance are required, hence, ease of maintenance is improved.

### Summary of the embodiments

Technical ideas understood from the embodiments will be described below citing the reference signs, etc., used for the embodiments. However, each reference sign, etc., described below is not intended to limit the constituent elements in the claims to the members, etc., specifically described in the embodiments.
(1) A blood purification device 1, comprising: a plurality of separate units 3, 4, wherein among the plurality of units 3, 4, at least one unit 3 comprises a storage unit 7 to store calibration data 91 of the unit 3, and when the unit 3 is connected to another unit 4, the calibration data 91 can be shared with the other unit 4.
(2) The blood purification device 1 as defined by (1), wherein the storage unit 7 stores operation data 92 including information related to operation of the unit 3, and the unit 3 with the storage unit 7 is configured to be able to share the operation data 92 with the other unit 4 when the unit 3 is connected to the other unit 4.
(3) The blood purification device 1 as defined by (2), wherein the unit 3 with the storage unit 7 comprises a control unit 6 that controls operation of the unit 3, and wherein the control unit 6 stores control contents as the operation data 92 in the storage unit 7.
(4) The blood purification device 1 as defined by any one of (1) to (3), comprising: as the plurality of units 3,4, a dialysate supply-and-discharge unit 3 that supplies a dialysate to a blood purifier 2 and discharges a waste liquid from the blood purifier 2, and an extracorporeal circulation unit 4 comprising an extracorporeal circulation part 41 to extracorporeally circulate blood of a patient through the blood purifier 2, wherein at least the dialysate supply-and-discharge unit 3 comprises the storage unit 7 to store the calibration data 91 of the own unit, and is configured to be able to share the calibration data 91 with the extracorporeal circulation unit 4 when the dialysate supply-and-discharge unit 3 and the extracorporeal circulation unit 4 are connected.
(5) A blood purification system 100, comprising: a blood purification device 1 comprising a plurality of separate units 3, 4; and a network device 101 that is configured to be able to communicate with at least one unit 4 among the plurality of units 3, 4 and comprises a storage unit 7 to store calibration data 91 of a predetermined unit 3 thereamong, wherein the at least one unit 4 of the blood purification device 1 comprises a control unit 6 that, when the unit 4 is connected to another unit 3, acquires the calibration data 91 of the connected other unit 3 through the network device 101.

Although the embodiments of the invention has been described, the invention according to claims is not to be limited to the embodiments described above. Further, please note that not all combinations of the features described in the embodiments are necessary to solve the problem of the invention. In addition, the invention can be appropriately modified and implemented without departing from the gist thereof.

### REFERENCE SIGNS LIST

1: blood purification device, 2: blood purifier, 3: dialysate supply-and-discharge unit, 4: extracorporeal circulation unit, 41: extracorporeal circulation part, 6: control unit, 61: first control unit, 62: second control unit, 7 storage unit, 71: first storage unit, 72: second storage unit, 81: operation control unit, 82: calibration processing unit, 83: connection checking unit, 91: calibration data, 92: operation data, 93: verification database, 100: blood purification system, 101: network device

## Claims

1. A blood purification device, comprising:
a plurality of separate units,
wherein among the plurality of units, at least one unit comprises a storage unit to store calibration data of the unit, and when the unit is connected to another unit, the calibration data can be shared with the other unit.

2. The blood purification device according to claim1, wherein the storage unit stores operation data including information related to operation of the unit, and the unit with the storage unit is configured to be able to share the operation data with the other unit when the unit is connected to the other unit.

3. The blood purification device according to claim 2, wherein the unit with the storage unit comprises a control unit that controls operation of the unit, and wherein the control unit stores control contents as the operation data in the storage unit.

4. The blood purification device according to claim 1, comprising:
as the plurality of units, a dialysate supply-and-discharge unit that supplies a dialysate to a blood purifier and discharges a waste liquid from the blood purifier, and an extracorporeal circulation unit comprising an extracorporeal circulation part to extracorporeally circulate blood of a patient through the blood purifier,
wherein at least the dialysate supply-and-discharge unit comprises the storage unit to store the calibration data of the own unit, and is configured to be able to share the calibration data with the extracorporeal circulation unit when the dialysate supply-and-discharge unit and the extracorporeal circulation unit are connected.

5. A blood purification system, comprising:
a blood purification device comprising a plurality of separate units; and
a network device that is configured to be able to communicate with at least one unit among the plurality of units and comprises a storage unit to store calibration data of a predetermined unit thereamong,
wherein the at least one unit of the blood purification device comprises a control unit that, when the unit is connected to another unit, acquires the calibration data of the connected other unit through the network device.
